# EUROPEAN PATENT APPLICATION

(11) **EP 0 791 357 A2**
(43) Date of publication of application: **27.08.1997**
(21) Application number: 96307351.5
(22) Date of filing: 09.10.1996
(51) Int. Cl.: A61K 38/01, C07K 14/415

(54) **Use of gluten peptides as stimulator of mineral absorption and preventive agent of hyperlipidermia and hypercholesterolemia**

(30) Priority: 21.02.1996 KR 9604070
(71) Applicant: Samyang Genex Co., Ltd., Chongno-Ku, Seoul 110-470 (KR)
(72) Inventor: Lee, Hyoun-Soo, Shinjong-Dong, Yangchon-Ku, Seoul (KR); Lee, Yun-Dong, Seo-ku, Taejon (KR); Bae, Chun-Ho, Yusong-Ku, Taejon (KR); Kim, Tae-Jong, Yusong-Ku, Taejon (KR); Lee, Yeon-Sook, Kunpo, Kyunggi-Do (KR)
(74) Representative: Woods, Geoffrey Corlett

(57) **Abstract**

Vegetable peptides, particularly gluten peptides, which have a molecular weight of from 200 to 6000 daltons and which comprise at least 20 mole% or more of acidic amino acids are used in the manufacture of a medicament for stimulating the intestinal absorption of a mineral such as calcium or iron or for preventing or treating hyperlipidemia and hypercholesterolemia. The peptides can be obtained by the enzymatic hydrolysis of a vegetable protein. Pharmaceutical compositions comprising the peptides, and a process for producing such compositions, are also described.

## Description

### Field of the Invention

The present invention relates to the novel use of gluten peptides which are prepared by the enzymatic hydrolysis of vegetable proteins, more specifically, to a stimulator of mineral absorption and a preventive agent of hyperlipidemia and hypercholesterolemia which comprises the gluten peptides as their active ingredients.

### Background of the Invention

Nutrition of dietary proteins have been chiefly emphasized in a view of their basic function as nitrogen source, amino acid supplier and metabolic intermediate as well. Naturally, dietary proteins have been simply evaluated in light of their quantity and quality of amino acids constituting the proteins.

However, nutrition of dietary protein has become newly highlightened for a decade, since other functions of peptides derived from the proteins, in addition to their basic function, have been successively reported in scientific societies. In this connection, several peptides derived from the proteins have been confirmed to have their own characteristic physiological functions, grounded on the findings that: many peptides derived from precursor proteins in the body, have physiological significance; and, primary structures common to the peptides also appear in the dietary proteins.

In conclusion, a variety of peptides having diverse molecular weight produced in the digestive organ during protein dissimilation have been proven to play various roles of physiological significance. For example, casein phosphopeptide("CPP") produced in the course of digestion of a milk protein(i.e., casein) has been reported to act as a stimulator of calcium absorption. Also, some peptides produced in digesting procedure have been reported to inhibit angiotensin converting enzyme(ACE) activity and make a function as opioide.

Further, peptides which can inhibit the degeneration of the mucous membrane of the small intestine and can inhibit angiotensin converting enzyme activity so that it can be used for the treatment of hyperpiesia, have been prepared by the enzymatic hydrolysis of a wheat protein, gluten or a corn protein, zein. Specifically, the peptides prepared from gluten, a byproduct of corn starch production, have been widely used as nutrient supplier, tube feeding, and protein supplement in the soy sauce or stock feed preparation.

On the other hand, calcium and iron, among the essential minerals to human body, have been known as nutrients apt to lack in the body. As the human life is enriched more and more, the intake amount of calories exceeds the daily recommended amount, which has become a causative factor for various geriatric diseases. Nevertheless, the intake amount of the calcium and iron is still under 90% of the daily recommended amount, which, in turn, causes porosis of bone and severe anemia. Accordingly, in order to supplement calcium and iron lacking in the body, they should be taken in an excessive amount, which naturally overstrains stomach to cause indigestion, etc. Under the circumstances, the way of stimulating their absorption rate has been explored, since it is more efficient than taking them excessively.

On the other hand, a meal therapy which is a method for the treatment of disease by intaking resticted diets, has become the subject of everyone's concern in the prevention and treatment of the geriatric disease which is the most common disease associated with the circulatory system. For example, high level of lipid and cholesterol in blood has attracted public attention as a primary cause of circulatory disorders such as brain trouble, arteriosclerosis, hyperpiesia, etc.; the meal therapy accompanying with regulation of meal uptake, however, can control the lipid and cholesterol level which are affected by the amount of cholesterol, type and amount of lipid, type of hydrocarbon, vitamin and mineral, etc. The close relationship between type of protein and lipid metabolism, even though the results are varied depending on the period of experiment, condition of experiment, and type and age of emperimental animal, has revealed that casein elevates cholesterol level in blood while soybean protein decreases.

As a proposed mechanism, changes in the lipid absorption rate depending on the type of protein and amino acid composition, lysine/arginine ratio, composition of sulfur-containing amino acids, and excretory amount of lipid and cholesterol in feces, have been suggested to make contribution in the regulation of lipid and cholesterol, though they are not clearly understood. Recently, proteins and peptides produced in the course of digestion, have been also proposed to interact with bile, lipid and cholesterol, etc. For example, high molecular weight peptides produced during digestion have been proposed, as a causative material in the reduction of lipid and cholesterol level by soybean protein. In conclusion, nutritional and physiological significance of dietary peptides have stressed the exploitation of novel peptides to be utilized in a form of foodstuffs or drugs.

Under the circumstances, the present inventors have made an effort to develop a novel peptides produced by the enzymatic hydrolysis of inexpensive and easily obtainable vegetable proteins.

In general, vegetable peptides have been prepared by the enzymatic hydrolysis of proteins from soybean, wheat and corn, etc.

For example, Japanese patent laid-open publication No. (Hei)3-204900 discloses peptide-containing substance which is prepared by the steps of: treating vegetable proteins with starch hydrolase; and, heating the material thus obtained under a highly alkaline condition and adding alkaline protease to remove aromatic amino acids. The said method requires an essential step of removing aromatic amino acids in order to remove a bitter taste and increase the relative content of branched amino acids which are known to improve the muscular movement. The prior art teaches that the peptide-containing substance can be used as nutrient supplier after hard exercise, illness, etc., since it can be absorbed rapidly into digestive tracks in an analogous manner as amino acids.

### Summary of the Invention

The present inventors have prepared peptides by the enzymatic hydrolysis of vegetable proteins which are byproducts of starch production, which have molecular weight of 200 to 6000 daltons and comprise 20 mole% or more acidic amino acids, and discovered that they stimulates intestinal absorption of calcium and iron and prevents hyperlipidemia and hypercholesterolemia.

The primary object of the present invention is, therefore, to provide a stimulator of mineral absorption comprising the peptides as active ingredient.

The other object of the invention is to provide a preventive agent of hyperlipidemia and hypercholesterolemia comprising the peptides as active ingredient.

### Brief Description of Drawings

The above and the other objects and features of the present invention will become apparent from the following descriptions given in conjunction with the accompanying drawings, in which:
- Figure 1: is a graph showing the weight of the rats fed with diets which comprise 10%(w/w) of gluten, gluten hydrolysate, the supernatant or precipitate of the gluten hydrolysate, in accordance with breeding time.
- Figure 2: is a graph showing the weight of the rats fed with diets which comprise 2%(w/w) of gluten, gluten hydrolysate, the supernatant or precipitate of the gluten hydrolysate, in accordance with breeding time.

### Detailed Description of the Invention

The present inventors prepared gluten peptides which have molecular weight of 200 to 6000 daltons and comprise 20 mole% or more acidic amino acids, by the enzymatic hydrolysis of the vegetable proteins such as corn and wheat gluten with protease such as papain and bromelain at 30 to 80°C, more preferably at 50 to 60°C under acidic to neutral environment of pH 4.0 to 8.0, most preferably pH 6.0, for 20 to 30 hours.

The gluten peptides thus prepared (hereafter referred to "gluten hydrolysate") were found to inhibit the precipitation of calcium and iron in vitro. In addition, it was revealed that the rats fed with the gluten hydrolysates had high concentration of soluble calcium in their small intestines, since the amount of precipitated calcium was low.

The present inventors, therefore, fed each group of rats with different diets in which gluten, gluten hydrolysate, the supernatant or precipitate of the gluten hydrolysate were added respectively as the nitrogen source, and investigated whether the gluten hydrolysate can stimulate intestinal absorption of calcium and iron in vivo. As a result, the experimental groups fed with the gluten hydrolysate and the supernatant of the gluten hydrolysate showed increased absorption rate of calcium and iron, while all experimental groups did not show any statistical significance in average weight, daily food intake, daily weight increment, and dietary efficiency through the starting and ending points of food supply. Also, it was found that absorption rate of calcium and iron increases as the concentration of gluten hydrolysate was increased, which clearly demonstrated that the gluten hydrolysate stimulates the intestinal absorption of calcium and iron. Further, it was also found that citric acid, sodium salt, potassium salt and calcium salt, etc. enhanced the inhibitory action of the gluten peptides on the precipitation of calcium and iron.

On the other hand, in order to investigate whether the gluten hydrolysate has a preventive effect of dietary fat-induced hyperlipidemia and hypercholesterolemia, mature rats were fed with experimental diets each of which were prepared to comprise casein, casein hydrolysate, gluten and gluten hydrolysate, respectively, in addition to basic diets containing high fat and high cholesterol, and the level of occurrence of hyperlipidemia and hypercholesterolemia was examined. Also, mature rats were fed with diets comprising high fat, high cholesterol and casein as protein source to make dietary fat-induced hyperlipidemia rats, and then they were fed with the said experimental diets to investigate their effect on the treatment of hyperlipidemia and hypercholesterolemia. In this experiment, arterial blood, liver, heart and feces were collected from each rat, and the contents of total lipids and cholesterol from each sample were measured.

A series of experiments carried out in the invention and results thereofs are summarized as follows:
1. The effect of gluten hydrolysate on the prevention of dietary fat-induced hyperlipidemia and hypercholesterolemia was investigated, which resulted that: food intake in the experimental groups was not different, and weight gain was relatively low in the rats fed with gluten or its hydrolysate. The effect of gluten hydrolysate on the treatment of dietary fat-induced hyperlipidemia and hypercholesterolemia was also investigated, which resulted that: both food intake and weight gain were not different in the experimental groups.
2. The rats fed with casein and gluten hydrolysate were observed to have the highest and the lowest value in the concentration of total serum lipids, respectively, and there were significant N-source effect and hydrolysate effect in the concentration of serum lipid, which suggest that gluten and its hydrolysate have hypolipidemic effect.
3. The effects of gluten and its hydrolysate intake on the concentration of total serum cholesterol were similar to those of total serum lipids. That is, there were significant N-source effect and hydrolysate effect in the concentration of serum cholesterol, which reveals that gluten and its hydrolysate have hypocholesterolemic effect. Also, it was found that intake of protein hydrolysate resulted in elevated level of serum HDL-cholesterol and the decreased level of serum LDL-cholesterol.
4. Intake of gluten or protein hydrolysate significantly lowers total lipids and cholesterol in liver, while it shows no effect in heart.
5. Intake of gluten significantly elevates the fecal excretion of total lipids and total cholesterol, while intake of its hydrolysate shows no effect.

Therefore, it was clearly demonstrated that gluten and its hydrolysate have hypolipidemic and hypocholesterolemic effects, that is, they can lower the concentration of serum lipids and the content of lipids in liver. Accordingly, gluten and its hydrolysate can be used for the prevention and treatment of dietary fat-induced hyperlipidemia and hypercholesterolemia as active ingredients of foodstuffs.

In accordance with the present invention, the gluten peptides which stimulate the intestinal absorption of calcium and iron and prevents hyperlipidemia and hypercholesterolemia, can be prepared from inexpensive and easily obtainable corn and wheat, which naturally guarantees that a mineral absorption stimulator and preventive agent of hyperlipidemia and hypercholesterolemia comprising them as active ingredients can be provided massively in a low price.

The mineral absorption stimulator and preventive agent of hyperlipidemia and hypercholesterolemia of the invention which comprise the gluten hydrolysate as an active ingredient, can be orally adminstrated or injected as formulation comprising pharmaceutically acceptable carriers.

For oral administration, the peptides can be formulated into a solid preparation such as tablets, pills, granules, powder, capsules and the like, or a liquid preparation such as solutions, suspensions, emulsions and the like. The pharmaceutical preparations for oral administration can contain active peptide or peptides alongside the customary excipients such as (a) fillers and extenders, for example starches, lactose, sucrose, glucose, mannitol and silica, (b) binders, for example carboxymethylcellulose, alginates, gelatine and polyvinylpyrrolidone, (c) humectants, for example glycerine, (d) disintegrating agents, for example agar-agar, calcium carbonate and sodium carbonate, (e) solution retarders, for example paraffin, (f) absorption accelerators, for example quaternary ammonium compound, (g) wetting agents, for example cetyl alcohol or glycerine monostearate, (h) adsorbents, for example kaolin and bentonite, (i) lubricants, for example talc, calcium stearate and magnesium stearate and solid polyethylene glycols, (j) colorants, (k) flavourings, (l) sweeteners, or mixtures of the substances listed under (a) to (l).

When the preparation is used for parental administration, the preparation is made in an injection formula, an intravenous drip infusion and the like. For the preparation of an injection formula, the solutions and emulsions can be in a sterile form which is isotonic with blood. The suspensions can contain in addition to the active peptide or peptides, preservatives, stabilizers, solubilisers, wetting agents, salts for changing the osmotic pressure or buffers. They can further contain other clinically useful substances.

The present invention is further illustrated in the following examples, which should not be taken to limit the scope of the invention.

### Example 1: Preparation of the gluten hydrolysate

To prepare the gluten hydrolysate, 1.5kg of corn gluten or wheat gluten was added to 1L of water, and incubated at 50°C with the addition of 10 to 150g of papain or bromelain under an environment of pH 6.0. During the reaction, the reactor was equipped with pH controller, by which constant pH was maintained by the addition of NaOH or HCl. 24 hours of reaction produces the gluten hydrolysate which comprises 20 mole% or more acidic amino acids, and possess molecular weight of 200 to 6000 daltons. The gluten hydrolysate was centrifuged, and the supernatant and the precipitate were obtained respectively and used in the following examples.

### Example 2: Feeding of rats with diet containing 10%(w/w) gluten hydrolysate

24 male Sprague-Dawley rats which were 4 weeks old were divided into 4 groups, and then fed for 4 weeks ad libitum with different diets which contains nitrogen source of 10%(w/w) gluten, gluten hydrolysate, the supernatant or precipitate of the gluten hydrolysate, respectively, in addition to 10%(w/w) soybean protein. The rats were separately raised in shoe-box cages in the room, while maintains the temperature of 22±2°C, relative humidity of 65±5% and light condition from a.m. 6:00 to p.m. 6:00. Also, semi-purified diets whose components and composition according to AIN-76(American Institute of Nutrition Standards for Nutritional Studies Report, J. Nutr., 107:1340-1348(1977)) were shown in Table 1 below, were used as experimental diets.

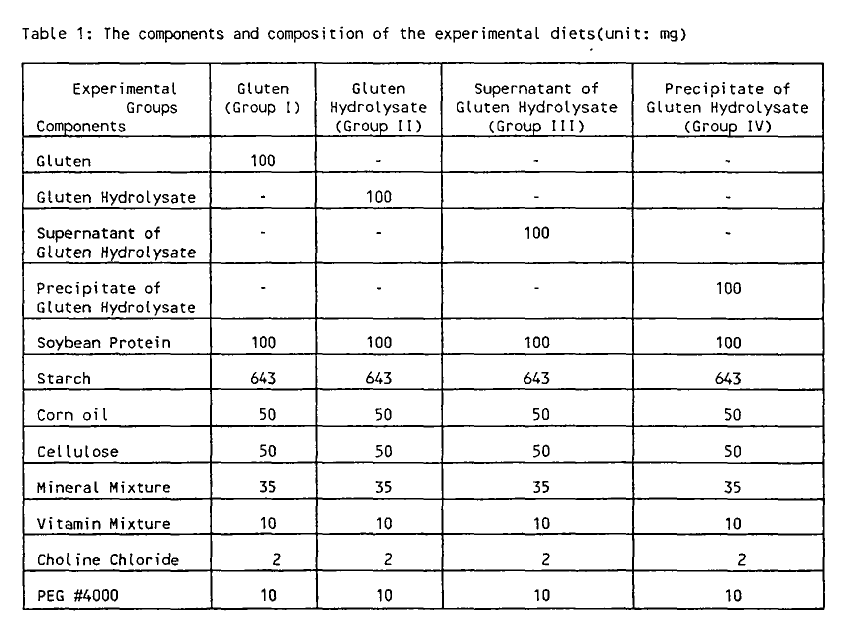

### Example 2-1: Measurement of the weight increment

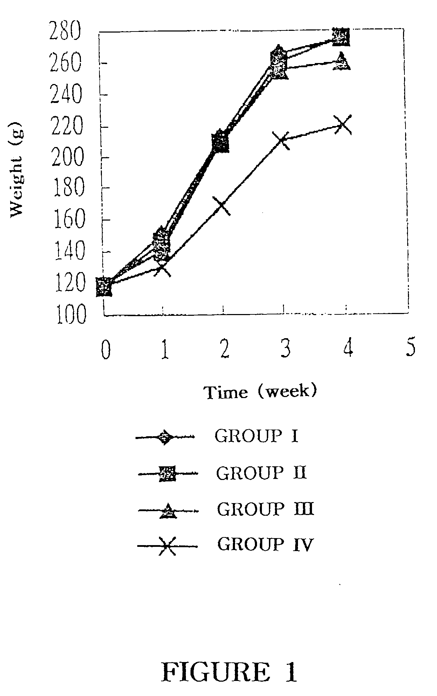

Daily food intake and weight increment of the rats were investigated. As a result, it was examined that all experimental groups showed no significant differences, while IV group showed a little reduction of the weight increment due to the reduction of the daily food intake(see: Figure 1).

### Example 2-2: The absorption rate of calcium and iron

The absorption rate of calcium and iron in the rats were investigated and summarized in Tables 2 and 3 below. The concentration of calcium was determined by the conventional method, and the concentration of iron was measured by the aid of a reagent for iron-quantitative analysis(Wako, Japan). In this experiment, the preserved amount(mg/dl or µg/ml) was determined by the amount of the daily food intake from which the amount of body wastes through feces and urine was subtracted, and the apparent absorption rate(mg/dl or µg/ml) was determined by the amount of daily food intake from which the amount of body wastes through feces was subtracted, respectively. And, the percentages of the preserved amount and the apparent absorption rate were calculated against the daily food intake.

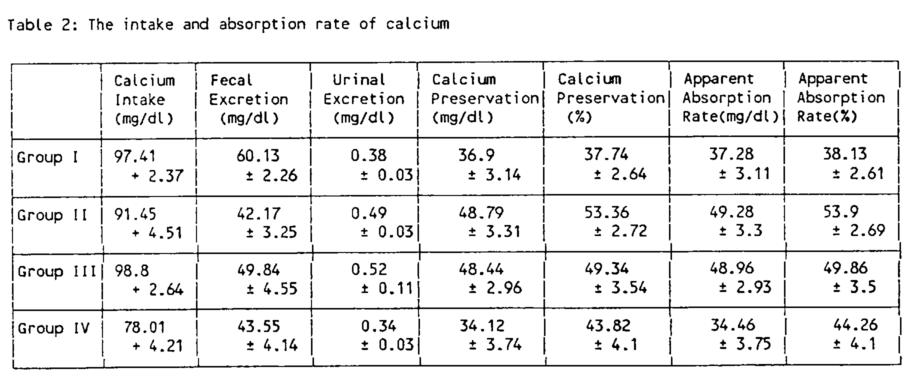

The apparent absorption rates of calcium(%) were compared. As can be seen in Table 2, group I fed with diet containing gluten was 38%, while group II fed with diet containing gluten hydrolysate was 54%, which corresponds the increase of apparent absorption rate by 42%. And, group III fed with diet containing the supernatant of gluten hydrolysate was 50%, which the increase of apparent absorption rate by 31%. Accordingly, it was clearly demonstrated that the gluten hydrolysate stimulates intestinal absorption of calcium.

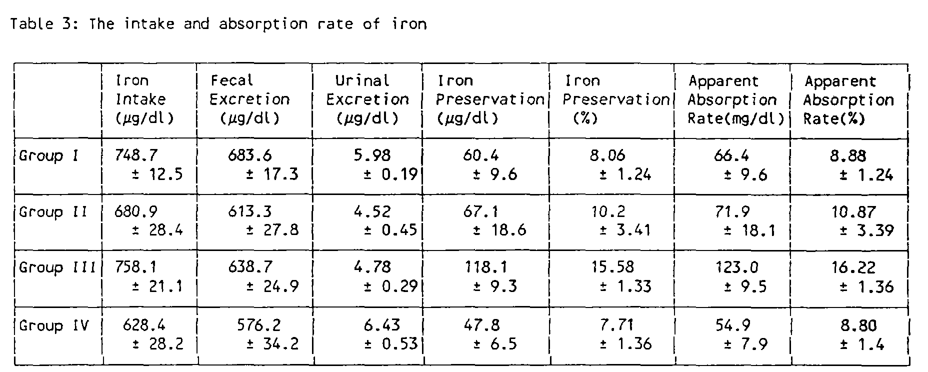

When the apparent absorption rates of iron(%) were compared, group I fed with diet containing gluten was 9%, while group III fed with diet containing the supernatant of gluten hydrolysate was 16%, which corresponds the increase of apparent absorption rate by 80%. Accordingly, it was also demonstrated that the gluten hydrolysate stimulates intestinal absorption of iron, and calcium as well.

### Example 3: Feeding of rats with diet containing 2%(w/w) gluten hydrolysate

18 male Sprague-Dawley rats which were 4 weeks old were divided into 3 groups, and then fed for 4 weeks ad libitum with different diets which contain nitrogen source of 2%(w/w) gluten, gluten hydrolysate or the supernatant of the gluten hydrolysate, respectively, in addition to 18%(w/w) soybean protein. The rats were raised in an analogous manner as in Example 2, and the components and composition of the experimental diets were shown in the Table 4 below.

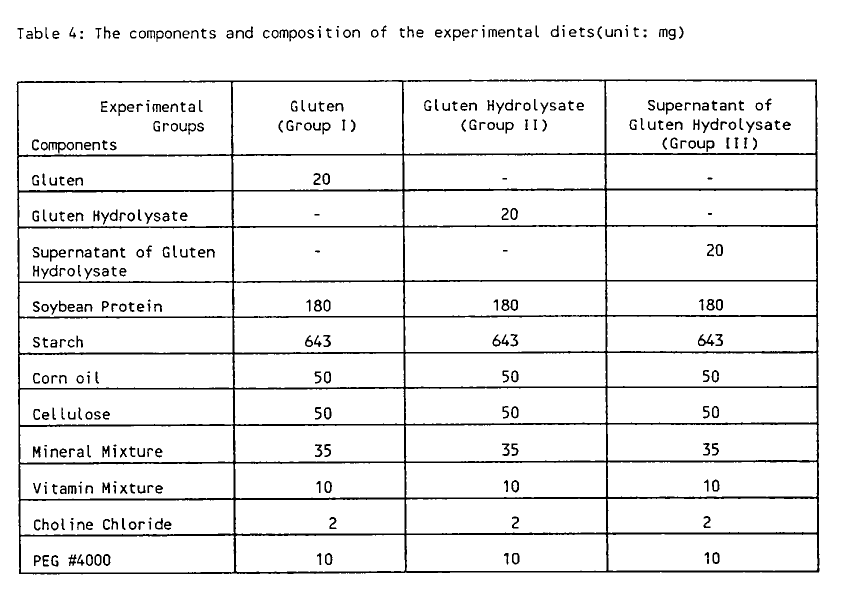

### Example 3-1: Measurement of the weight increment

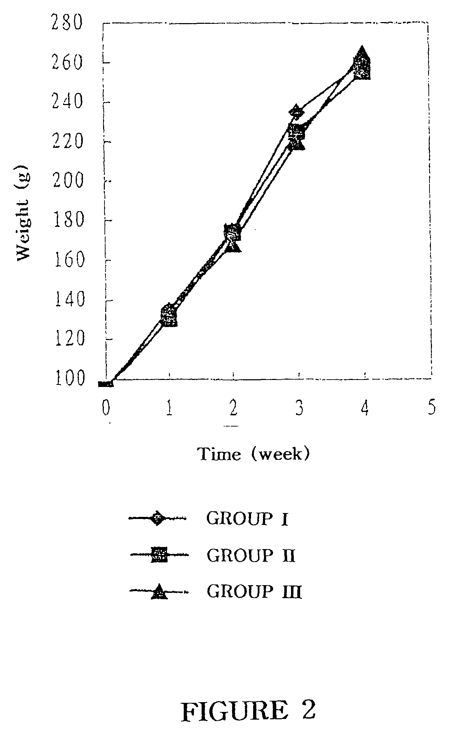

The average weight, daily food intake, daily weight increment, and dietary efficiency during feeding were examined. As a result, the group fed with the supernatent of gluten hydrolysate showed somewhat high average weight in the ending points of food supply, while all experimental groups did not show any statistical significance (see: Figure 2).

### Example 3-2: The absorption rate of calcium and iron

The absorption rate of calcium and iron in the rats were investigated and summarized in Tables 5 and 6 below. The concentrations of calcium and iron were determined in a manner analogous to that described in Example 2-2. In this experiment, the preserved amount(mg/dl or µg/ml), the apparent absorption rate(mg/dl or µg/ml), and their percentages, were calculated analogously as in Example 2-2.

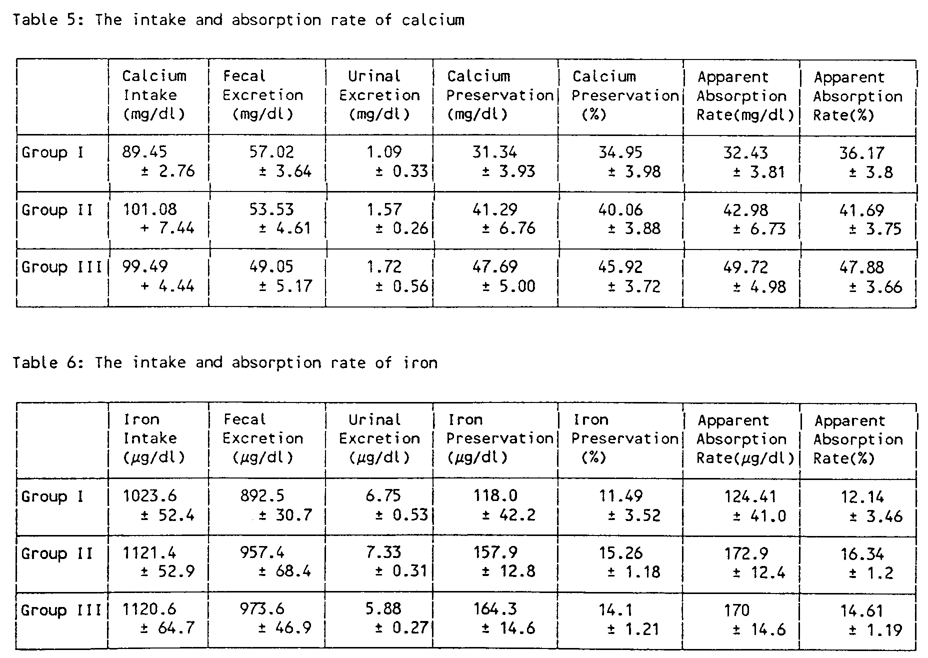

The apparent absorption rates of calcium and iron(%) in Tables 5 and 6 were compared with those in Tables 2 and 3. As a result, the group fed with 2%(w/w) supernatant of gluten hydrolysate was lower than that of the group fed with 10%(w/w) supernatant of gluten hydrolysate, but the group was higher than the group fed with 2% gluten by the increase of 10 to 20%.

### Example 4: Enhancement of the inhibition of calcium precipitation by citric acid or NaCl

In order to enhance the inhibition of calcium precipitation by the gluten hydrolysate, 0.01M citric acid, malic acid or NaCl was added to 0.01%(w/v) gluten hydrolysate, reacted for 1 to 3 hours at 30 to 80°C, and then powdered by spray drying. The powder thus prepared was dissolved in 0.2ml of distilled water. Then, 1ml of 0.01M CaCl₂.2H₂O and 2ml of 0.02M phosphate buffer(pH 7.0) were added to the dissolved powder, incubated at 37°C for 1 hour, and centrifuged at 12,000rpm for 5 minutes employing a small-scale centrifuge equipped with a 0.1µm ultrafiltration membrane to obtain the supernatant. And then, the concentrations of calcium in the supernatants were determined, respectively(see: Table 7).

**Table 7**

| The amount of the soluble calcium | |
|---|---|
| Sample | Relative Recovery of Calcium(%) |
| Gluten Hydrolysate | 100 |
| Gluten Hydrolysate + 0.01M citric acid | 150 |
| Gluten Hydrolysate + 0.01M malic acid | 90 |
| Gluten Hydrolysate + 0.01M NaCl | 130 |

As shown in Table 7 above, it was revealed that the inhibition of the calcium precipitation by the gluten hydrolysate was enhanced by the addition of citric acid or NaCl.

### Example 5: Effect of gluten hydrolysate on the prevention of hyperlipidemia and hypercholesterolemia

32 male Sprague-Dawley rats which had average weight of 160g were randomly divided into 4 groups, and fed for 6 weeks ad libitum with experimental diets, each of which comprises casein(C), casein hydrolysate(CH), gluten(G) and gluten hydrolysate(GH) were added, respectively, in addition to basic diets comprising high fat(18% in diet) and high cholesterol (1% in diet), in order to investigate the level of occurrence of hyperlipidemia and hypercholesterolemia. The rats were raised in an analogous manner as in Example 2, metabolic experiments were carried out for four days before the sacrifice of rats, and semi-purified diets whose components and composition were shown in Table 8 below, were used as experimental diets. The experimental diets were prepared according to AIN-76, with the exception of adding beef tallow(Seoul Agricultural Products Co., Korea, purified) in a concentration of 18% which is high level of fat compared to the standard concentration of fat, 5% according to NAS-NRC(National Research Council, Nutrient Requirements of Laboratory Animals No.10, NAS, Washington, D.C.(1972)). Further, egg protein(Shinwoo Chemical Co., Korea) having the protein content of 82% was added in a concentration of 10% as nitrogen source. Also, casein(Maeil Milk Co., Ltd., Korea), casein hydrolysate(Maeil Milk Co., Ltd., Korea), gluten and gluten hydrolysate were added in a concentration of 10% in different diets, respectively. All experimental diets have the same content of nitrogen, and comprise 1% of PEG(polyethylene glycol) #4000 as a marker for movement of diet within the digestive tract.

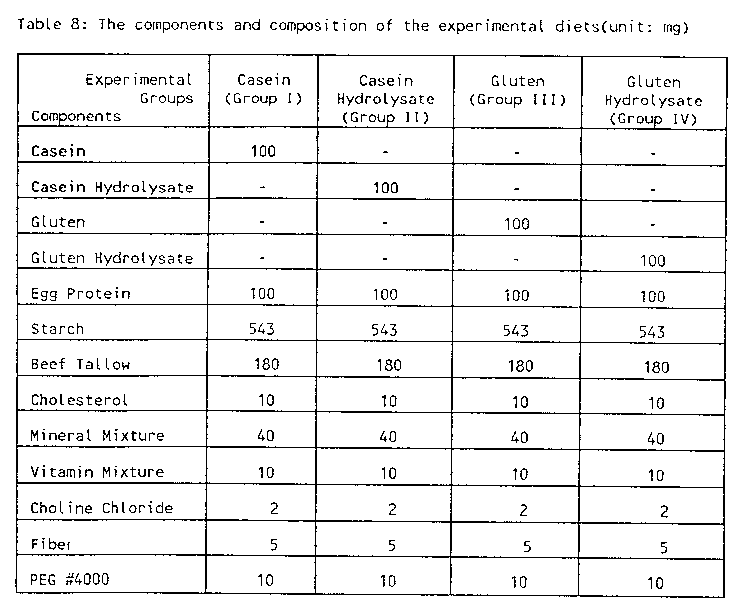

Food supply to the experimental animals was stopped for 12 hours before collection of samples, and then was taken for 1.5 hours. After 1 hour from the completion of food supply, the rats were anesthetized with ethylether, and blood was collected from the carotid. Then, liver and heart were collected, fat attached to them was removed, they were washed with cold physiological saline(0.9% NaCl solution) to remove blood, their weights were measured, and their feces were collected for four days before the sacrifice of rats. Blood thus collected was stored in the refrigerator(4°C) for 24 hours, and centrifuged at 3,000rpm for 20 minutes to obtain serum. The tissues and the feces were freeze-dried employing Freeze-Dryer 18(Labconco, USA), ground, and then their dry weights were measured. All samples were stored below -40°C until they were used for analysis.

### Example 5-1: Body weight and food intake

Body weight, weight gain, food intake and feed efficiency in the rats fed with experimental diets were measured and shown in Table 9 below. In this experiment, weight gain divided by food intake gives feed efficiency. As can be seen in Table 9, final body weight, weight gain and feed efficiency showed significant differences among the experimental groups, while food intake in the groups were not significantly different. The rats fed with casein and casein hydrolysate showed higher weight gain than those fed with gluten and gluten hydrolysate, which may be caused by the difference of amino acid composition.

Experimental data was analyzed employing SAS program and represented as average and standard error(mean±SE). The significant difference, the N-source(type of protein) effect [(C+CH):(G+GH)] and the hydrolysate effect[(C+G):(CH+GH)] were examined employing Dumcan's multiple range test(p<0.05).

**Table 9**

| Body weight, food intake and feed efficiency in the rats fed with experimental diets | | | | | |
|---|---|---|---|---|---|
| Experimental Group | Initial Body Weight(g) | Final Body Weight(g) | Weight Gain (g/day) | Food Intake (g/day) | Feed Efficiency |
| Group I(C) | 166.0±4.6^{NS}¹⁾ | 393.3±14.6^{a}²⁾ | 5.41±0.31^{a} | 17.48±0.53^{NS}³⁾ | 0.31 ± 0.01^{a} |
| Group II(CH) | 165.1±3.3 | 385.3±10.2^{a} | 5.26±0.24^{a} | 18.44±1.01 | 0.26 ± 0.02^{b} |
| Group III(G) | 165.3±4.6 | 365.0±10.9^{ab} | 4.76±0.18^{ab} | 17.73±0.71 | 0.27 ± 0.01^{b} |
| Group IV(GH) | 164.9±4.2 | 348.2±8.0^{b} | 4.36±0.12^{b} | 17.67±0.23 | 0.25 ± 0.01^{b} |
| N-Source Effect | NS | P<0.05 | P<0.05 | NS | P<0.05 |
| Hydrolysate Effect | NS | NS | NS | NS | P<0.05 |

| | | | | | |
|---|---|---|---|---|---|
| 1) Mean ± SE | | | | | |
| 2) Values with different superscript within the column are significantly different at p<0.05. | | | | | |
| 3) NS: not significantly different | | | | | |

### Example 5-2: The concentration of total lipids and neutral fats in serum

The concentration of total lipids and neutral fats were measured in the sera of the rats fed with experimental diets and shown in Table 10 below. In this experiment, the concentration of total lipids in serum was measured by the colorimetric method employing sulfophospho vanillin reaction according to Fringe and Dunn's method(see: Fringe, C.S. and Dunn, R.M., Am. J. Clin. Patho., 53:89-92(1980)), and the concentration of triglyceride in serum was measured according to Biggs et al.'s method(see: Biggs et al., Clin. Chem., 21:437-441(1975)).

**Table 10**

| The concentration of total lipids and triglyceride in serum | | |
|---|---|---|
| Experimental Group | Total lipids (mg/100ml) | Triglyceride (mg/100ml) |
| Group I(C) | 324.1±31.3^{a} (100%)¹⁾ | 37.5±3.3^{NS} (100%) |
| Group II(CH) | 250.2±13.8^{b} (70%) | 31.7±4.1 (80%) |
| Group III(G) | 245.2±24.4^{b} (76%) | 34.0±2.5 (91%) |
| Group IV(GH) | 190.1±19.5^{b} (59%) | 31.9±3.8 (85%) |
| N-Source Effect | P<0.05 | NS |
| Hydrolysate Effect | P<0.05 | NS |

| | | |
|---|---|---|
| 1) Parentheses represent percentage(%) against the casein diet group. | | |

As can be seen in Table 10, the concentration of total serum lipids showed significant differences among the experimental groups, while the concentration of serum triglyceride in the groups were not significantly different. The concentration of total serum lipid was observed to be significantly higher in the rats fed with casein than other groups among which the concentration of total serum lipid was not significantly different. Particularly, the concentration of total serum lipid was observed to be the lowest value in the rats fed with gluten hydrolysate, i.e., the decreased value of 40% compared with the group fed with casein and the decreased value of 22% compared with the group fed with gluten.

On the other hand, it was found that there was significant N-source effect in the concentration of serum lipid where the effect of gluten and its hydrolysate had the value of 74% compared with one of the effect of casein and its hydrolysate. This result consists with previous reports that the concentration of serum lipid was significantly lower in the group fed with soy protein than the group fed with casein. Also, it was found that there was significant hydrolysate effect in the concentration of serum lipid where the effect of protein hydrolysate had the value of 75% compared with one of the effect of protein. Accordingly, it was concluded that gluten hydrolysate had a preventive effect of dietary fat-induced hyperlipidemia.

### Example 5-3: The concentration of total cholesterol, HDL-cholesterol and LDL-cholesterol in serum

The concentration of total cholesterol and HDL-cholesterol in serum were measured, LDL-cholesterol in serum and athero-index were calculated according to Friedwald's equation, and they were shown in Table 11 below. In this experiment, the concentration of serum cholesterol was measured according to Zlatkis and Zak's method(see: Zlatkis, A. and Zak, B., Anal. Biochem., 29:143-146(1969)), and the concentration of serum HDL-cholesterol was measured employing the enzymatic kit(Youngdong Pharm. Co., Ltd., Korea).

**Table 11**

| The concentration of total cholesterol, HDL-cholesterol, LDL-cholesterol and Athero-Index in serum | | | | |
|---|---|---|---|---|
| Experimental Group | Cholesterol (mg/100ml) | | | Athero-Index²⁾ |
| | Total | HDL | LDL¹⁾ | |
| Group I (C) | 122.6±6.9^{a} (100%) | 25.8±2.1^{b} (100%) | 89.5±6.5^{a} (100%) | 3.65±0.34^{a} (100%) |
| Group II (CH) | 104.2±3.9^{b} (85%) | 33.4±3.7^{ab} (129%) | 65.7±6.3^{b} (73%) | 2.17±0.28^{b} (60%) |
| Group III (G) | 111.8±2.3^{ab} (91%) | 25.1±1.9^{b} (97%) | 77.7±1.8^{ab} (87%) | 3.48±0.29^{a} (95%) |
| Group IV (GH) | 104.1±4.8^{b} (85%) | 36.1±2.7^{a} (140%) | 64.5±5.5^{b} (72%) | 1.91±0.21^{b} (52%) |
| N-Source Effect | NS | NS | NS | NS |
| Hydrolysate Effect | P<0.05 | P<0.05 | P<0.05 | P<0.05 |

| | | | | |
|---|---|---|---|---|
| 1) LDL-cholesterol: Total cholesterol-(HDL-cholesterol)-Triglyceride/5 | | | | |
| 2) Athero-Index: (Total cholesterol-(HDL-cholesterol))/(HDL-cholesterol) | | | | |

As shown in Table 11, the concentration of total serum cholesterol showed significant difference between the groups fed with protein and its hydrolysate, respectively, while showed no difference according to the type of protein. Particularly, the rats fed with casein hydrolysate showed significant reduction of 15% in the concentration of total serum cholesterol compared with the group fed with casein. The rats fed with gluten hydrolysate showed reduction in the concentration of total serum cholesterol compared with the group fed with gluten, though it did not show any significant difference. Also, there was no significant difference in the concentration of total serum cholesterol between the groups fed with casein and gluten, respectively.

On the other hand, the rats fed with protein hydrolysate showed significant high value in the concentration of total serum HDL-cholesterol compared with the group fed with protein(p<0.05), and particularly, the rats fed with gluten hydrolysate showed increase of 40% or more in the concentration of total serum HDL-cholesterol compared with the group fed with casein or gluten. Therefore, it was assumed that intake of protein hydrolysates, inter alia, gluten hydrolysate, results in the reduction in occurrence of diseases associated with the circulatory system such as atherosclerosis and hyperpiesia, since HDL-cholesterol has been known to show the inhibitory effect on the detrimental factors of the diseases.

Moreover, the rats fed with protein hydrolysate showed significant reduction compared with the group fed with protein in the concentration of serum LDL-cholesterol which was calculated employing total serum cholesterol, serum HDL-cholesterol and triglyceride(p<0.05). And, the rats fed with gluten showed reduction in the concentration of serum LDL-cholesterol compared with the group fed with casein, though it did not show any significant difference.

Accordingly, it was demonstrated that intake of protein hydrolysate lowers total serum cholesterol and elevates serum HDL-cholesterol, which finally leads to significant decrease of athero-index.

### Example 5-4: The contents of total lipids, total cholesterol and triglyceride in liver

Wet and dry weight of liver, and the contents of total lipids, total cholesterol and triglyceride in liver were measured and shown in Table 12. The total fat in liver was extracted by Folch et al.'s method(see: Folch et al., J. Biol. Chem., 226:497-502(1957)), and the contents of total cholesterol and triglyceride in liver were determined in an analogous manner as in Example 5-2.

**Table 12**

| The contents of total lipids, total cholesterol and triglyceride in liver | | | | | |
|---|---|---|---|---|---|
| Experimental Group | Weight(g) | | Total Lipids (mg/dry wt.g) | Total Cholesterol (mg/dry wt.g) | Triglyceride (mg/dry wt.g) |
| | Wet Tissue (g) | Dry Tissue (g) | | | |
| Group I(C) | 17.6±0.8^{a} | 8.46±1.23^{a} | 541.6±12.7^{a} | 30.3±1.4^{a} | 105.6±4.0^{a} |
| Group II(CH) | 17.5±0.9^{a} | 8.21±0.37^{a} | 496.4±10.6^{b} | 26.8±1.2^{ab} | 83.1±7.9^{b} |
| Group III(G) | 15.6±0.9^{ab} | 6.81±0.47^{b} | 491.2±8.6^{b} | 26.1±1.0^{b} | 81.8±4.8^{b} |
| Group IV(GH) | 14.7±0.4^{b} | 6.24±0.19^{b} | 456.9±10.6^{c} | 24.0±1.4^{b} | 71.8±7.0^{b} |
| N-Source Effect | P<0.05 | P<0.05 | P<0.05 | P<0.05 | P<0.05 |
| Hydrolysate Effect | NS | NS | P<0.05 | NS | P<0.05 |

As can be seen in Table 12, wet weight and dry weight of liver, and the contents of total lipids, total cholesterol and triglyceride in liver showed significant differences among the experimental groups. The weight of liver was observed to be significantly higher in the rats fed with casein than the group fed with gluten, while the weight of liver between the groups fed with protein and protein hydrolysate, respectively, was not significantly different. The contents of total lipids, total cholesterol and triglyceride in liver were significantly lower in the group fed with gluten than the group fed with casein. The contents of total lipids and triglyceride in liver were lower in the group fed with protein hydrolysate than the group fed with protein, though it did not show any significant difference. Therefore, it could be concluded that the type of protein and intake of protein hydrolysate make an effect on the lipid metabolism in the liver.

Accordingly, it was demonstrated that the lipid metabolism in the liver is influenced by the type of protein and the nitrogen source, that is, intake of protein hydrolysate or gluten lowers the lipid content in the liver.

### Example 5-5: The contents of total lipids, total cholesterol and triglyceride in heart

Wet and dry weight of liver, and the contents of total lipids, total cholesterol and triglyceride in heart were measured in an analogous manner as in Example 5-4 and summarized in Table 13.

**Table 13**

| The contents of total lipids, total cholesterol and triglyceride in heart | | | | | |
|---|---|---|---|---|---|
| Experimental Group | Weight(g) | | Total Lipids (mg/dry wt.g) | Total Cholesterol (mg/dry wt.g) | Triglyceride (mg/dry wt.g) |
| | Wet Tissue (g) | Dry Tissue (g) | | | |
| Group I(C) | 1.23±0.07^{NS} | 0.28±0.02^{NS} | 133.3±5.2^{a} | 14.7±0.5^{NS} | 8.05 ± 0.87^{a} |
| Group II(CH) | 1.25±0.05 | 0.28±0.01 | 122.9±2.8^{ab} | 13.9±0.4 | 4.01 ± 0.27^{b} |
| Group III(G) | 1.19±0.05 | 0.25±0.01 | 131.9±4.2^{a} | 14.5±0.3 | 7.05 ± 1.04^{a} |
| Group IV(GH) | 1.16±0.05 | 0.26±0.02 | 116.6±3.1^{b} | 13.7±0.4 | 3.32 ± 0.53^{b} |
| N-Source Effect | NS | NS | NS | NS | NS |
| Hydrolysate Effect | NS | NS | P<0.05 | NS | P<0.05 |

As can be seen in Table 13, the weight of heart and the content of total cholesterol showed no significant differences among the experimental groups, and the contents of total lipids and triglyceride in heart showed significant differences among the experimental groups. That is, the type of protein did not result in the significant difference, while the contents of total lipids and triglyceride in heart were significantly lower in the rats fed with protein hydrolysate than the group fed with protein(p<0.05). Particularly, the contents of total lipids were significantly low in the group fed with gluten hydrolysate. The contents of triglyceride in heart was lower in the group fed with each protein hydrolysate than the group fed with each protein.

### Example 5-6: The fecal excretion of total lipids

The weight of feces, and fecal excretion of total lipids, total cholesterol and triglyceride in the experimental groups were measured and shown in Table 14. The contents of cholesterol and triglyceride were determined in the same manner as in Example 5-4.

**Tabel 14**

| The fecal excretion of total lipids, total cholesterol and triglyceride | | | | |
|---|---|---|---|---|
| Experimental Group | Dry Weight (g/day) | Total Lipids (mg/day) | Total Cholesterol (mg/day) | Triglyceride (mg/day) |
| Group I(C) | 1.61±0.12^{NS} | 181.7±10.9^{b} | 15.6±1.3^{NS} | 1.73±0.21^{NS} |
| Group II(CH) | 1.81±0.17 | 228.9±12.5^{a} | 17.0±1.4 | 1.99±0.44 |
| Group III(G) | 1.72±0.13 | 224.5±14.5^{a} | 19.4±1.1 | 1.79±0.09 |
| Group IV(GH) | 1.51±0.06 | 221.7±8.8^{a} | 18.9±1.4 | 1.69±0.10 |
| N-Source Effect | NS | NS | P<0.05 | NS |
| Hydrolysate Effect | NS | NS | NS | NS |

As can be seen in Table 14, the weight of feces showed no significant differences among the experimental groups. Only the fecal excretion of total lipids showed significant differences among the experimental groups, that is, it was low in the rats fed with casein compared with the other groups. Intake of gluten resulted in significant high fecal excretion of total cholesterol and total lipids. The rats fed with protein hydrolysate showed increasing tendency in the fecal excretion of total lipids, total cholesterol and triglyceride compared with the group fed with intact protein, though it did not show any significant difference.

Accordingly, it was demonstrated that intake of gluten has increasing effect of fecal excretion of total lipids, total cholesterol and triglyceride.

### Example 6: Effect of gluten hydrolysate on the treatment of hyperlipidemia and hypercholesterolemia

32 male Sprague-Dawley rats which had average weight of 160g were fed for 4 weeks with diets comprising high fat, high cholesterol and casein as protein source to make dietary fat-induced hyperlipidemia rats. And then, they were randomly divided into 4 groups, and fed for 4 weeks ad libitum with experimental diets as in Example 5 in order to investigate the effect on the treatment of hyperlipidemia and hypercholesterolemia. In this connection, the components and composition of the experimental diets, the breeding condition, the collection of samples, the analysis of the samples and the statistical analysis were carried out as described in Example 5.

### Example 6-1: Body weight and food intake

Body weight, weight gain, food intake and feed efficiency in the rats fed with experimental diets were measured and shown in Table 15 below. As shown in Table 15, body weight and food intake showed no differences among the experimental groups, and there were no N-source effect and no hydrolysate effect.

**Table 15**

| Body weight, food intake and feed efficiency in the rats fed with experimental diets | | | | | |
|---|---|---|---|---|---|
| Experimental Group | Initial Body Weight(g) | Final Body Weight(g) | Weight Gain (g/day) | Food Intake (g/day) | Feed Efficiency |
| Group I(C) | 164.8±4.0^{NS} | 413.7±12.1^{NS} | 4.45±0.21^{NS} | 16.40±0.58^{NS} | 0.27±0.01^{NS} |
| Group II(CH) | 165.9±5.6 | 403.7±13.5 | 4.25±0.14 | 15.17±0.56 | 0.28±0.02 |
| Group III(G) | 165.5±4.7 | 414.2±14.8 | 4.42±0.19 | 16.11±0.57 | 0.27±0.01 |
| Group IV(GH) | 164.3±4.1 | 416.6±16.6 | 4.44+0.28 | 15.96±0.87 | 0.25±0.02 |
| N-Source Effect | NS | NS | NS | NS | NS |
| Hydrolysate Effect | NS | NS | NS | NS | NS |

### Example 6-2: The concentration of total lipids and neutral fats in serum

The concentration of total lipids and neutral fats were measured in the sera of the rats fed with experimental diets and shown in Table 16 below.

**Table 16**

| The concentration of total lipids and triglyceride in serum | | |
|---|---|---|
| Experimental Group | Total lipids (mg/100ml) | Triglyceride (mg/100ml) |
| Group I(C) | 333.8±25.5^{a} (100%)¹⁾ | 42.3±3.9^{NS} (100%) |
| Group II(CH) | 244.0±22.9^{b} (73%) | 38.6±4.7 (91%) |
| Group III(G) | 232.8±29.7^{b} (71%) | 37.0±6.3 (88%) |
| Group IV(GH) | 187.8±13.9^{b} (57%) | 35.7±5.1 (84%) |
| N-Source Effect | p<0.05 | NS |
| Hydrolysate Effect | p<0.05 | NS |

| | | |
|---|---|---|
| 1) Parentheses represent percentage(%) against the casein diet group. | | |

As can be seen in Table 16, the concentration of serum triglyceride showed no significant differences among the experimental groups. That is, it was not influenced by type of protein and nitrogen source. But the group fed with casein had the highest value in the concentration of total serum lipids, and the group fed with gluten hydrolysate had the lowest value, which consists with the result in Example 5-2. However, the result in Example 5-2 where the rats were fed with the experimental diets for 6 weeks from the beginning must be evaluated differently with one in the present Example where in the aspect of the treatment, the rats were fed with the experimental diets for 4 weeks after they were fed with fat-enriched diet comprising casein for 4 weeks to induce hyperlipidemia.

Accordingly, it was demonstrated that gluten hydrolysate had a effect of treatment of dietary fat-induced hyperlipidemia since intake of gluten or its hydrolysate resulted in reduction in the concentration of total serum lipid.

### Example 6-3: The concentration of total cholesterol, HDL-cholesterol and LDL-cholesterol in serum

The concentration of total cholesterol and HDL-cholesterol in serum were measured, LDL-cholesterol in serum and athero-index were calculated according to Friedwald's equation, and they were shown in Table 17 below.

**Table 17**

| The concentration of total cholesterol, HDL-cholesterol, LDL-cholesterol and Athero-Index in serum | | | | |
|---|---|---|---|---|
| Experimental Group | Cholesterol (mg/100ml) | | | Athero-Index |
| | Total | HDL | LDL | |
| Group I(C) | 195.3±17.9^{a} (100%) | 33.7±4.9^{NS} (100%) | 126.5±10.9^{a} (100%) | 2.90±0.88^{NS} (100%) |
| Group II(CH) | 134.6±10.5^{b} (69%) | 38.7±3.9 (115%) | 87.2±34.4^{ab} (69%) | 2.46±0.84^{a} (85%) |
| Group III(G) | 125.9±8.9^{b} (64%) | 34.8±4.8 (103%) | 82.5±10.3^{b} (65%) | 2.97±0.93^{a} (102%) |
| Group IV(GH) | 106.5±11.8^{b} (55%) | 47.3±6.0 (140%) | 54.5±15.2^{b} (43%) | 1.90±0.23^{a} (66%) |
| N-Source Effect | p<0.05 | NS | NS | NS |
| Hydrolysate Effect | NS | NS | NS | NS |

As shown in Table 17, the group fed with casein had significantly high value in the concentration of total serum cholesterol, and the group fed with gluten hydrolysate had the lowest value. The group fed with gluten or protein hydrolysate showed reduction to 55% to 69% in the concentration of total serum cholesterol compared with the group fed with casein, which suggests that gluten hydrolysate can be used for the treatment of dietary fat-induced hypercholesterolemia.

The rats fed with gluten hydrolysate showed increase of 40% or more in the concentration of total serum HDL-cholesterol compared with the group fed with casein or gluten. Therefore, it was assumed that intake of protein hydrolysate, inter alia, gluten hydrolysate, results in the reduction in occurrence of diseases associated with the circulatory system such as atherosclerosis and high pressure, since HDL-cholesterol has been known to have the inhibitory effect on the detrimental factors of the diseases.

In addition, the rats fed with protein hydrolysate showed decreasing tendency in the concentration of serum LDL-cholesterol compared with the group fed with intact protein, though it did not show any significant difference. Also, the rats fed with gluten showed decreasing tendency in the concentration of serum LDL-cholesterol compared with the group fed with casein, though it did not show any significant difference.

Accordingly, it was demonstrated that intake of protein hydrolysate lowers total serum cholesterol and elevates serum HDL-cholesterol, which leads to significant decrease of athero-index.

### Example 6-4: The contents of total lipids, total cholesterol and triglyceride in liver

Wet weight and dry weight of liver, and the contents of total lipids, total cholesterol and triglyceride in liver were measured and shown in Table 18.

**Table 18**

| The contents of total lipids, total cholesterol and triglyceride in liver | | | | | |
|---|---|---|---|---|---|
| Experimental Group | Weight(g) | | Total Lipids (mg/dry wt.g) | Total Cholesterol (mg/dry wt.g) | Triglyceride (mg/dry wt.g) |
| | Wet Tissue (g) | Dry Tissue (g) | | | |
| Group I(C) | 20.3±1.1^{NS} | 10.4±0.82^{NS} | 553.2±5.9^{a} | 35.1±2.3^{a} | 123.4±12.3^{a} |
| Group II(CH) | 19.0±1.0 | 9.1±0.62 | 494.6±17.8^{b} | 26.2±1.0^{b} | 84.4±5.8^{b} |
| Group III(G) | 21.9±1.4 | 10.1±0.68 | 550.7±12.4^{a} | 28.7±1.3^{b} | 128.3±21.6^{a} |
| Group IV(GH) | 19.5±1.3 | 9.3±0.58 | 514.9±18.4^{ab} | 28.5±1.2^{b} | 83.8±7.1^{b} |
| N-Source Effect | NS | NS | NS | NS | NS |
| Hydrolysate Effect | NS | NS | p<0.05 | p<0.05 | p<0.05 |

As can be seen in Table 18, the body weight of liver showed no significant differences among the experimental groups, and the contents of total lipids, total cholesterol and triglyceride in liver showed significant differences, where their levels were significantly low in the rats fed with protein hydrolysate compared with the group fed with the intact protein, and there was no difference according to the type of protein.

Accordingly, it was demonstrated that intake of protein hydrolysate results in a decreasing effect of total lipids, total cholesterol and triglyceride in liver.

### Example 6-5: The contents of total lipids, total cholesterol and triglyceride in heart

Wet weight and dry weight of liver, and the contents of total lipids, total cholesterol and triglyceride in heart were measured and shown in Table 19.

**Table 19**

| The contents of total lipids, total cholesterol and triglyceride in heart | | | | | |
|---|---|---|---|---|---|
| Experimental Group | Weight(g) | | Total Lipids (mg/dry wt.g) | Total Cholesterol (mg/dry wt.g) | Triglyceride (mg/dry wt.g) |
| | Wet Tissue (g) | Dry Tissue (g) | | | |
| Group I(C) | 1.31±0.04^{NS} | 0.28±0.1^{NS} | 126.3±5.0^{NS} | 12.6±0.8^{NS} | 11.85±1.45^{a} |
| Group II(CH) | 1.35±0.06 | 0.28±0.01 | 120.6±4.3 | 11.7±1.0 | 12.24±2.39^{a} |
| Group III(G) | 1.35±0.04 | 0.29±0.01 | 118.6±2.7 | 12.7±0.5 | 6.66±0.69^{b} |
| Group IV(GH) | 1.46±0.07 | 0.30±0.01 | 113.4±4.1 | 11.3±1.1 | 6.24±1.01^{b} |
| N-Source Effect | NS | NS | NS | NS | p<0.05 |
| Hydrolysate Effect | NS | NS | NS | NS | NS |

As can be seen in Table 19, only the content of triglyceride in heart showed significant differences among the experimental groups. That is, the contents of triglyceride in heart were significantly lower in the rats fed with gluten than the group fed with casein. Therefore, it was suggested that the type of protein does not influence the contents of total lipids in heart, and the hydrolysate effect can not be expected by the intake for 4 weeks.

### Example 6-6: The fecal excretion of total lipids

The weight of feces, and fecal excretion of total lipids, total cholesterol and triglyceride in the experimental groups were measured and shown in Table 20.

**Table 20**

| The fecal excretion of total lipids, total cholesterol and triglyceride | | | | |
|---|---|---|---|---|
| Experimental Group | Dry weight (g/day) | Total lipids (mg/day) | Total Cholesterol (mg/day) | Triglyceride (mg/day) |
| Group I(C) | 1.40±0.07^{NS} | 165.6±5.1^{b} | 16.0±2.0^{NS} | 0.88±0.11^{c} |
| Group II(CH) | 1.48±0.09 | 185.7±19.8^{ab} | 16.2±2.0 | 1.56±0.16^{b} |
| Group III(G) | 1.39±0.05 | 193.0±8.0^{ab} | 18.6±1.9 | 2.02±0.23^{b} |
| Group IV(GH) | 1.61±0.08 | 217.2±14.1^{a} | 18.3±1.2 | 2.62±0.26^{a} |
| N-Source Effect | NS | p<0.05 | NS | p<0.05 |
| Hydrolysate Effect | NS | NS | NS | p<0.05 |

As can be seen in Table 20, the fecal excretion of total lipids and triglyceride showed significant differences among the experimental groups, that is, they had the lowest values in the rats fed with casein and the highest values in the rats fed with gluten hydrolysate. Further, they showed significant differences according to the type of protein, that is, they were higher in the group fed with gluten or its hydrolysate than the group fed with casein or its hydrolysate. Also, the rats fed with gluten showed increasing tendency in the fecal excretion of total cholesterol compared with the group fed with casein, though it did not show any significant difference.

Accordingly, it was demonstrated that intake of gluten has Increasing effect of fecal excretion of total lipids, total cholesterol and triglyceride.

### Safety test of the gluten hydrolysate

The gluten hydrolysate of the present invention was orally administrated to 4 week-old female and male Sprague-Dawley rats with a maximized dose of 10g/kg body weight. As a result, no harmful effect was detected.

As clearly illustrated and demonstrated above, the present invention provides a stimulator of mineral absorption and a preventive agent of hyperlipidemia and hypercholesterolemia comprising peptides prepared by the enzymatic hydrolysis of vegetable proteins, which have molecular weight of 200 to 6000 daltons, as their active ingredients and comprise 20 mole% or more acidic amino acids. In accordance with the present invention, it was determined the gluten peptides can be used for the development of a stimulator of the intestinal absorption of calcium and iron and preventive agent of dietary fat-induced hyperlipidemia and hypercholesterolemia in a form of foodstuff.

## Claims

1. Use of a vegetable peptide which has a molecular weight of from 200 to 6000 daltons and which comprises at least 20 mole% of acidic amino acids in the manufacture of a medicament for stimulating the intestinal absorption of a mineral or for preventing or treating hyperlipidemia or hypercholesterolemia.

2. Use according to claim 1, wherein the medicament is for stimulating the intestinal absorption of calcium or iron.

3. Use according to claim 1 or 2 wherein the medicament further comprises one or more substances selected from citric acid, a sodium salt, a potassium salt and a calcium salt.

4. A vegetable peptide which has a molecular weight of from 200 to 6000 daltons and which comprises at least 20 mole % of acidic amino acids for use in a method of treatment of the human or animal body by therapy or prophylaxis.

5. A vegetable peptide as claimed in claim 4 for use in stimulating the intestinal absorption of a mineral or in preventing or treating hyperlipidemia or hypercholesterolemia.

6. A pharmaceutical composition which comprises a pharmaceutically acceptable carrier or diluent and, as an active ingredient, a vegetable peptide which has a molecular weight of from 200 to 6000 daltons and which comprises at least 20 mole% of acidic amino acids.

7. A process for producing a composition as claimed in claim 6, which process comprises subjecting to enzymatic hydrolysis a vegetable protein selected from corn gluten, wheat gluten and corn zein, and formulating the resulting vegetable peptide with a pharmaceutically acceptable carrier or diluent.

8. A process according to claim 7 wherein the enzymatic hydrolysis is carried out at a temperature of from 30 to 80° and a pH of from 4.0 to 8.0 for 20 to 30 hours.

9. A process according to claim 7 to 8 wherein the enzyme used in the enzymatic hydrolysis is papain or bromelain.

10. A vegetable peptide which has a molecular weight of from 200 to 6000 daltons and which comprises at least 20 mole% of acidic amino acids.
